# FASCICULE DE BREVET EUROPEEN

(11) **EP 0 937 063 B1**
(45) Date de publication et mention de la délivrance du brevet: **10.04.2002**
(21) Numéro de dépôt: 97913236.2
(22) Date de dépôt: 03.11.1997
(51) Int. Cl.: C07D 317/66, C07D 317/58, C07C 217/58, C07C 217/84, A61K 31/19, A61K 31/335

(54) **DERIVES ARYLOXYPROPANOLAMINES, LEUR PROCEDE DE PREPARATION ET LEURS APPLICATIONS**
ARYLOXYPROPANOLAMINDERIVATE, VERFAHREN ZU DEREN HERSTELLUNG UND DEREN VERWENDUNGEN
ARYLOXYPROPANOLAMINE DERIVATIVES, METHOD OF PREPARATION AND APPLICATIONS THEREOF

(30) Priorité: 05.11.1996 FR 9613438
(43) Date de publication de la demande: 25.08.1999
(73) Titulaire: VIRBAC S.A., F-06516 Carros Cedex (FR)
(72) Inventeur: EL HADRI, Ahmed, F-91240 Saint Michel sur Orge (FR); LECLERC, Gérard, F-38000 Grenoble (FR); STROSBERG, Arthur, Donny, F-75015 Paris (FR); PIETRI-ROUXEL, France, F-75014 Paris (FR); ARCHIMBAULT, Philippe, MC-98000 (MC)
(74) Mandataire: Mouget-Goniot, Claire
(86) Numéro de dépôt international: FR9701963
(87) Numéro de publication internationale: WO9820005

(56) Documents cités:
- EP-A- 0 300 290
- EP-A- 0 714 883

## Description

La présente invention est relative à des dérivés aryloxypropanolamines possédant au moins une activité anti-diabétique et anti-obésité ainsi qu'à leurs procédés de préparation et à leurs applications notamment comme médicaments en médecine humaine et vétérinaire et comme additif alimentaire animal.

Il existe deux formes majeures de diabète : le diabète de type I, insulino-dépendant, qui résulte d'une déficience complète en insuline et le diabète de type II ou insulino-indépendant, qui apparaît souvent en présence de taux normaux ou même légèrement élevés d'insuline et qui semble être le résultat d'une incapacité des tissus à répondre de manière appropriée à la présence d'insuline, due à des anomalies métaboliques dans la production et l'utilisation du glucose ; de telles anomalies empêchent le maintien d'un taux de glucose sanguin physiologique ; il en résulte une hyperglycémie. La plupart des diabétiques de type II sont également obèses.

Les méthodes habituelles de traitement du diabète de type II et de l'obésité comprennent, outre des régimes particuliers, l'utilisation d'agents β-adrénergiques et plus particulièrement l'utilisation d'agents β₃-adrénergiques (agonistes), qui stimulent la lipolyse.

Les composés qui stimulent les récepteurs β₃-adrénergiques présentent également une activité anti-obésité. De plus, ils présentent une activité hypo-glycémique (anti-hyperglycémique) et anti-diabétique, mais le mécanisme de cet effet ne semble pas connu.

C'est ainsi que pour le traitement du diabète ou de l'obésité, il a déjà été proposé d'utiliser de l'acide [p-[2[[(S)-2-hydroxy-3-phénoxypropanol]amino]éthyl]phénoxy acétique ou l'un de ses sels (Brevet européen 0 300 290) ou bien encore des dérivés d'aryloxypropanolamine dont la fonction amine est substituée par un groupement comportant deux cycles aromatiques éventuellement substitués (Brevet européen 0 714 883).

Parmi les produits présentant une activité (β₃-adrénergique, on peut citer :
- des phényléthanolamines (Brevet US 4,478,849 au nom d'Ainsworth et al. ; Brevet US 5,106,867 au nom de Bloom et al.; article publié dans Drugs Fut. 1993, 18(6) 541), qui sont considérés comme ayant une activité anti-obésité, anti-diabétique et anti-hyperglycémique ;
- des phénoxypropanolamines substituées à l'azote par des groupes éthers du type aryloxyalkyl (Brevets européens 0 210 849 et 0 254 532), qui sont considérées comme ayant essentiellement une activité anti-obésité ; des phénoxypropanolamines substituées par un phénylsulfonamide, relié au groupe amine par l'un des groupes suivants : -CH₂-, CH₂-CH₂-, CH=CH ou CH₂O- (Brevet européen 0 611 003), qui sont considérées comme ayant une activité dans le traitement du diabète de type II, de l'obésité et en tant qu'agents antidépresseurs ; des phénoxypropanolamines substituées par un groupe 1,3-benzodioxole-2,2-acide dicarboxylique (Brevet US 5,488,064), relié au groupe amine par le groupe suivant : 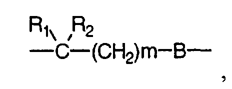
dans lequel B est une liaison ou un atome d'oxygène, m est égal à 1 ou à 2, R₁ et R₂ identiques ou différents, représentent un atome d'hydrogène ou un groupe alkyle inférieur, qui sont considérées comme ayant, outre une activité anti-diabétique, une activité anti-obésité, une activité sur les désordres de l'hypermotilité intestinale, sur la dépression et le stress, sur la régulation de la pression intraoculaire, l'hypertriglycéridémie, l'hypercholestérolémie, l'athérosclérose et les maladies cardiovasculaires.

En conséquence, les composés précités ont l'inconvénient majeur de ne pas être sélectifs vis-à-vis du récepteur β3-adrénergique ; ils peuvent donc présenter une activité vis-à-vis des récepteurs β1 et/ou β2 adrénergiques ; en particulier, le large spectre d'activité des composés décrits dans le Brevet US 5,488,064 peut conduire à des effets secondaires défavorables au niveau respiratoire et/ou cardiaque.

En conséquence, la Demanderesse s'est donné pour but de pourvoir à des composés présentant une activité β3-adrénergique sélective, qui répondent mieux aux besoins de la pratique que les composés de l'art antérieur.

En effet, un composé qui stimule de manière sélective les récepteurs β3-adrénergiques, c'est-à-dire qui a peu ou pas d'effets β1 ou β2, aura l'activité anti-diabétique et/ou anti-obésité recherchée, sans les effets indésirables liés à une stimulation concomitante β1 (augmentation de la fréquence cardiaque) ou β2 (tremblements musculaires).

La sélectivité de tels composés peut être déterminée en effectuant des études de liaison sur des cellules CHO-K1 β3 bovin, réalisées selon le protocole décrit dans Eur. J. Biochem., 1995, 230, 350-358 (F. Pietri-Rouxel et al.) ; les produits sont testés à une concentration maximale de 10 µMolaire sur les cellules entières, pour leur capacité à stimuler une accumulation d'AMPc (agoniste) ou à inhiber l'accumulation stimulée par une concentration (10 nM) d'isoprotérénol (antagoniste).

La présente invention a pour objet des composés qui répondent à la formule générale I suivante : 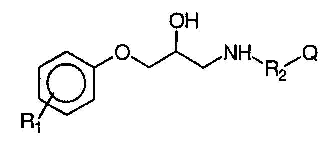 dans laquelle :
R₁, substituant du groupe phényle, en position 2, 3 ou 4, représente un atome d'hydrogène, un atome d'halogène ou l'un des groupes suivants : hydroxyle, alkyle inférieur en C₁-C₁₀, notamment choisi parmi les groupes méthyle, éthyle, propyle, isopropyle, butyle, tertiobutyle ; alkyloxy en C₁-C₁₀ et notamment méthoxy ; benzyloxy ; nitro ; cyano ; trifluorométhyle ; amino éventuellement substitué (mono ou di-substitué) par 1 ou 2 radicaux alkyles inférieurs, tels que précisés ci-dessus,
R₂ représente l'un des groupes suivants : -CH₂-, -CH₂-CH₂-, -CH(CH₃)-CH₂-, -C(CH₃)=CH-, -C(CH₃)₂-CH₂- ou une liaison ;
Q représente :
   (i) un radical phényle 3,4-disubstitué alkylène dioxy déterminant avec le radical phényle, un motif benzodioxane non substitué, un motif benzodioxol non substitué, ou un motif benzodioxol 2-substitué par deux radicaux R₃ et R₄, conformément à la formule suivante : 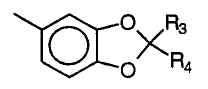 dans laquelle R₃ et R₄ sont sélectionnés de manière indépendante dans le groupe constitué par un atome d'hydrogène ou un groupe hydroxyméthyle,
   (ii) un radical phényle 3 et/ou 4-substitué, conformément à la formule II suivante 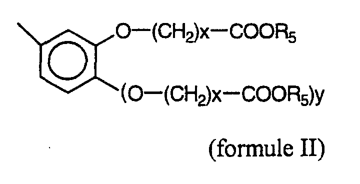 dans laquelle
      R₅ représente un atome d'hydrogène ou un radical alkyle inférieur en C₁-C₆, linéaire ou ramifié,
      x est un nombre entier compris entre 1 et 3
      y est 0 ou 1,
   (iii) un hydrocarbure polycyclique condensé comprenant au moins deux cycles condensés et choisis parmi les motifs suivants : indène, indacène, naphtalène, azulène, biphénylène, acénaphtylène, fluorène, phénalène, phénanthrène, anthracène,
   (iv) un système hydrocarboné cyclique, éventuellement ponté, et constituant un cycloalkane comprenant 1, 2 ou 3 cycles, comportant éventuellement des substituants choisis parmi les groupes méthyle, éthyle, isopropyle, propyle, butyle, tertiobutyle, le cycle principal comportant 5 ou 6 chaînons.

A titre d'exemple, on entend par cycloalkane à un cycle, un cyclopentane ou un cyclohexane ; par cycloalkane à deux cycles, les terpènes et par cycloalkane à trois cycles, notamment les adamantanes.

Les composés de formule (I) comprennent également les sels pharmacologiquement acceptables de ces dérivés ainsi que l'ensemble des isomères optiques et leurs composants ou mélanges de diastéréoisomères. Les produits basiques selon la présente invention peuvent être salifiés par les acides minéraux habituels et également par les acides organiques couramment employés comme tartrique, maléique, malonique, fumarique, succinique, méthanesulfonique, éthènesulfonique, hydroxyéthanesulfonique. Les dérivés à caractère acide peuvent être salifiés par les bases minérales pharmacologiquement acceptables comme les agents alcalins ou alcalino-terreux, ou par les bases organiques non toxiques comme les amines aliphatiques, les aminoalcools ou analogues.

Parmi les composés de formule (I) préférés, on peut citer :
- les composés comprenant un benzodioxol non substitué :
   ⇒ répondant à la formule suivante : 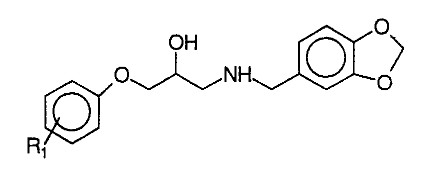 dans laquelle R₁ représente un atome d'hydrogène ; un méthoxy ou un atome de chlore en position para ; un atome de chlore, un groupe hydroxy, un groupe nitro, un groupe cyano ou un groupe trifluorométhyle en position méta ; ou
   ⇒ répondant à la formule suivante : 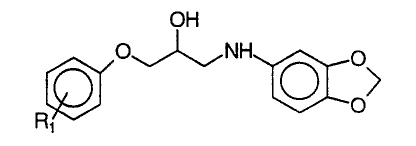 dans laquelle R₁ représente un atome d'hydrogène ou un groupe méthoxy en para ;
- le composé répondant à la formule suivante : 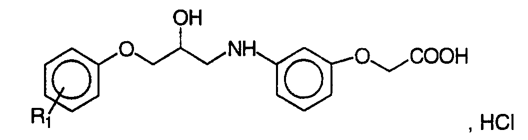 dans laquelle R₁ représente un atome d'hydrogène ;
- le composé répondant à la formule suivante : 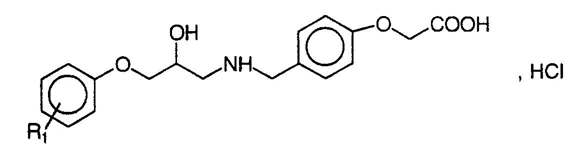 dans laquelle R₁ représente un atome d'hydrogène ; et
- les composés dans lesquels :
   R₁ représente un atome d'hydrogène, un 4-O méthyle, un 3-OH, un groupe trifluorométhyle,
   R₂ représente une liaison, ou l'un des groupes suivants : -CH₂- ou-CH₂-CH₂- et
   Q représente un groupe fluorène ou un système hydrocarboné cyclique, par exemple un cyclohexyle, un groupe adamantyl-1 ou un groupe adamantyl-2.

Les composés de formule (I) peuvent être préparés par réaction :
- d'un composé phénoxyépoxypropane de formule III 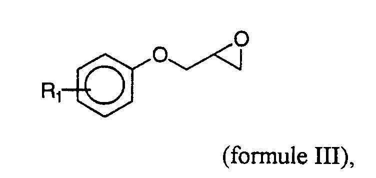 dans laquelle R₁ a la signification ci-dessus, avec
- une amine primaire répondant à la formule (IV) 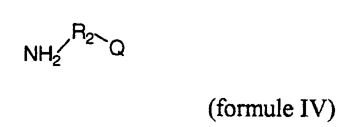
dans laquelle R₂ et Q ont les significations ci-dessus.

Certains composés de formule (III) sont accessibles commercialement ; sinon ils peuvent être préparés par réaction d'un phénol convenablement choisi quant à l'identité de R₁, avec une épichlorhydrine ou une épibromhydrine, en présence d'un accepteur d'halogène dans un solvant polaire, de préférence. Si la nature du substituant R₁ le rend sensible à cette réaction de condensation, il est préférable de le protéger avant la réaction, selon une technique adaptée comme décrit par exemple dans *"Protective Group in Organic Synthesis,* 2nd, Ed., T.W. Greeneand P.G.M. Wuts, John Wiley and sons, New York, 1991".

Certains composés selon la formule (IV) sont commerciaux ; d'autres peuvent être préparés par différentes méthodes, dont celle représentée ci-après, pour ce qui concerne le produit de formule (I) dans laquelle Q représente un groupement selon la formule (II).

Le procédé de préparation est illustré au schéma 1 ci-après.

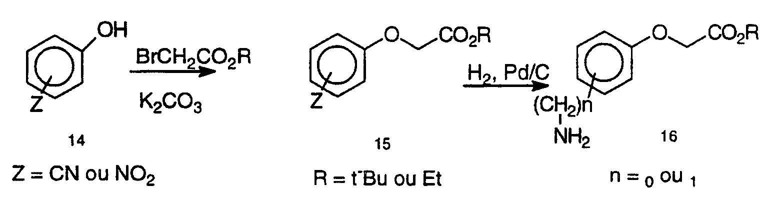

Le traitement du nitrophénol (ou du cyanophénol) 14 avec du tert-butylbromoacétate et du carbonate de potassium anhydre dans de l'acétone donne le produit 15 qui est soumis à une hydrogénation en présence de Pd/C(10 %) et qui conduit à l'amine 16 (schéma 1). Lorsque Z est un groupe cyano, la réduction catalytique en présence de Pd/C produit des dérivés mono- et dibenzylamine.

Les intermédiaires de formule (III) et de formule (IV) NH₂-R₂-Q sont couplés par chauffage (sous forme brut ou en solution dans un solvant polaire tel que le diméthylformamide anhydre), pendant 12 à 14 heures, à une température comprise entre 70 et 100°C, pour fournir des composés de formule (I). Certains composés de formule (I) sont obtenus avec une ou deux fonctions esters, qui réagissent respectivement avec HCl 6N à 80°C pendant 12 heures et avec NaOH 1N à température ambiante pendant 12 à 48 heures, pour obtenir le sel d'acide carboxylique comme illustré au schéma 2 :

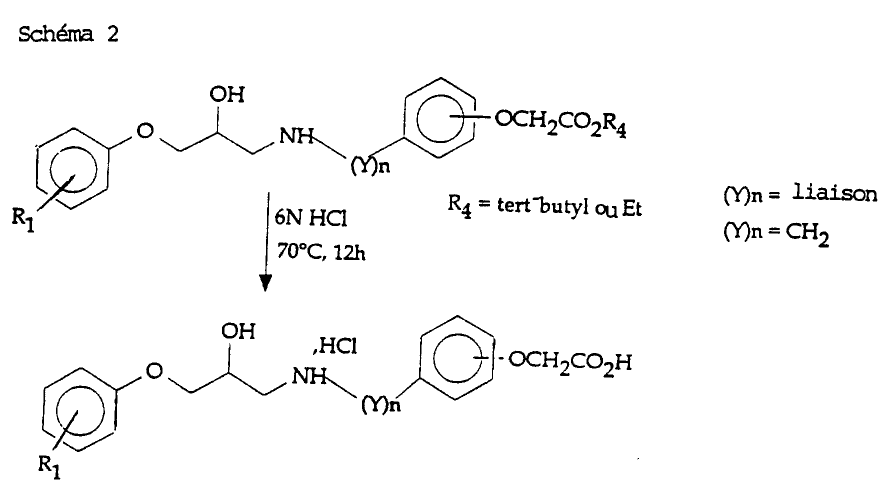

Les composés de formule (I) ont une activité agoniste particulièrement intéressante sur les récepteurs β3 adrénergiques : ils possèdent, en particulier, une action intéressante sur la thermogenèse et peuvent donc être utilisés dans le traitement de l'obésité ou dans les troubles du fonctionnement métabolique. Ils peuvent, en outre, dans certains cas aussi, modifier le catabolisme des graisses et être utilisés dans l'élevage animal, améliorant la production des muscles au détriment des graisses.

Ces composés peuvent être employés en alimentation animale pour éviter un engraissement non fructueux des animaux et favoriser, au contraire, l'accroissement de la masse musculaire.

Leur activité β3 se manifestant essentiellement sur le muscle lisse, la modération de la contraction intestinale ne se fait donc pas au détriment d'un effet cardio-vasculaire.

Une préparation contenant au moins un produit répondant à la formule (I) selon l'invention peut être administrée à l'animal, dont l'homme, atteint de diabète, d'obésité ou de dérèglement de la motilité intestinale.

Les produits selon l'invention sont administrés de préférence par voie orale ou sublinguale, mais d'autres voies d'administration peuvent aussi bien être utilisées (voies intranasale, transdermale, parentérale telles que sous-cutanée, intraveineuse, intrapéritonéale).

Les doses administrées, varient, selon le cas, de 0,1 à 100 mg/kg de poids vif, de préférence de 1 à 10 mg/kg de poids vif.

Si nécessaire, les composés selon la formule (I) peuvent être administrés en combinaison avec des produits β1 ou β2 adrénergiques.

Dans le traitement de l'obésité, éventuellement associé à un traitement du diabète, ces produits sont administrés à des doses de 1 à 10 mg/kg de poids corporel ; dans le cas de l'administration à l'animal des formes pharmaceutiques propres peuvent être employées suivant l'espèce et comporte notamment les solutions à étaler sur le corps de l'animal (solution à étaler manuellement, solution à étaler par vaporisation etc..).

Les composés de la présente invention sont formulés en comprimés, capsules, gélules ou sirop destinés à l'administration par voie orale. Ces formes gélules, capsules, comprimés peuvent contenir des excipients habituellement utilisés en formulation pharmaceutique tels que des adjuvants ou liants comme des amidons, des gommes, de la gélatine ainsi que des adjuvants comme le phosphate de calcium, des délitants comme l'amidon de maïs ou les acides alginiques, un lubrifiant comme le stéarate de magnésium, des agents édulcorants ou des arômes. Les solutions ou suspensions peuvent être préparées en milieu aqueux ou non aqueux par l'addition de solvants pharmacologiquement compatibles. Parmi ceux-ci, les glycols, polyglycols, propylène-glycols, éther de polyglycol, DMSO et éthanol.

Ils peuvent également être formulés en suspension ou solution stérile destinée à l'administration parentérale ou intranasale ou encore être présentés en patch transdermique. La dose unitaire comprise entre 1 et 500 mg de composé de formule (I) est mélangée avec un excipient adapté à la forme réalisée ; solvant, diluant, excipient, conservateur, édulcorant, parfum. La quantité de principe actif présent dans chaque dose unitaire est telle qu'elle puisse être administrée une ou plusieurs fois dans la journée. Des formulations à relargage programmé peuvent être également préparées pour obtenir un effet durable dans le temps permettant d'espacer les prises de traitement.

Les exemples cités illustrent le contenu de l'invention sans en limiter la portée aux seuls exemples décrits.

### EXEMPLE 1

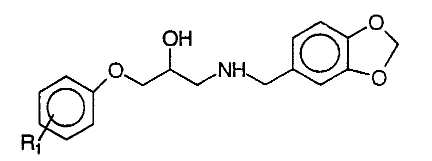

### N-((1,3-benzodioxole-5-yl)méthyl)-2-hydroxy-3-(3-hydroxyphénoxy)propylamine 1e

36,6 g (332,40 mmoles) de résorcinol et 42 g (331,78 mmoles) de benzylchlorure sont mis à reflux pendant une nuit sous agitation, en présence de 46,6 g de K₂CO₃ dans 250 ml de Me₂CO sec. Le mélange est refroidi, filtré et évaporé. Le résidu obtenu est dilué dans 500 ml d'eau et extrait à l'éthanol. La couche organique est lavée avec NaOH aqueux à 10 % et extraite à l'éthanol. L'huile de couleur rouge obtenue est distillée à l'aide d'un "appareil de distillation Kugelrohr" pour obtenir 18 g de m-PhCH₂OC₆H₄OH, b₁₅ 240-245°C (litt. b₁₁ 202-210°C). Une solution de 3,3 g (16,5 mmoles) de m-PhCH₂OC₆H₄OH dans 50 ml de DMF est traitée avec 0,8 g (16,5 mmoles) d'hydrure de sodium et le mélange est agité pendant 15 minutes. Après l'addition de 13,75 ml (16,5 mmoles) d'épichlorhydrine, le mélange est agité à 60°C pendant une heure jusqu'à complète alkylation. Le réactif en excès et le solvant sont évaporés sous pression réduite et le résidu est réparti entre les deux phases suivantes : de l'éthylacétate et de l'eau. La phase organique fournit l'époxyde, qui est utilisé sans autre purification.

L'époxyde est dissous dans du DMF contenant un équivalent d'amine et chauffé une nuit à 80°C. Après refroidissement, le solvant est éliminé sous vide et le résidu huileux est chromatographié sur une colonne de gel de silice en utilisant un mélange de solvants et recristallisé comme indiqué au Tableau I.

**TABLEAU I**

| **Données chimiques des composés I** | | | | |
|---|---|---|---|---|
| **Composé** | **R**_{**1**} | **Rdt %** | **Solvant de cristallisation** | **Pt de fusion °C** |
| I1a | H | 67 | MeOH | 102-103 |
| I1b | p-OCH₃ | 80 | MeOH | 107-108 |
| | | | | |
| I1c | m-Cl | 62 | MeOH | 128-129 |
| I1d | p-Cl | 74 | EtOH | 98-99 |
| I1e | m-OH | 62 | CH₃CN | 230-231 |
| I1f | m-NO₂ | 47 | MeOH | 129-130 |
| | | | | |
| I1g | m-CN | 55 | MeOH | 138-139 |
| I1h | m-CF₃ | 39 | MeOH | 121-122 |

**Les spectres RMN de** ^{**1**}**H (δ) des composés selon l'exemple 1 sont illustrés ci-après :**
**Composé 1a** (base libre), δ (ppm) : 2,59 (m, 2H), 3,62 (s, 2H), 3,90 (m, 3H), 5,95 (s, 2H), 6,78 (m, 2H Ar), 6,90 (m, 4H Ar), 7,26 (dt, 2H Ar, J = 7,73, 1,02 Hz).
**Composé 1b** (base libre), δ (ppm) : 2,52 (m, 2H), 3,61 (s, 2H), 3,68 (s, 3H), 3,83 (m, 3H), 4,88 (b, NH), 5,95 (s, 2H), 6,75 (dd, 1H Ar, J = 8,0, 1,38 Hz), 6,81 (d, 1H Ar, J = 8,0 Hz), 6,83 (s, 4H), 6,89 (d, 1H Ar, J = 1,1 Hz).
**Composé 1c** (maléate), δ (ppm) : 2,90 (dd, 1H, J = 13,56, 9,40 Hz), 3,07 (dd, 1H, J = 12,59, 2,94 Hz), 3,97 (d, 2H, J = 5,07 Hz), 4,10 (s, 2H), 4,12 (m, 1H), 6,01 (s, 2H), 6,04 (s, 2H), 6,90 (dd, 1H Ar, J = 8,31, 2,14 Hz), 7,00 (m, 4H Ar), 7,09 (s, 1H Ar), 7,31 (t, 1H Ar, J = 8,42 Hz).
**Composé 1d** (maléate), δ (ppm) : 2,90 (dd, 1H, J = 12,63, 9,20 Hz), 3,08 (dd, 1H, J = 12,67, 3,18 Hz), 3,93 (d, 2H, J = 5,18 Hz), 4,10 (s, 2H), 4,12 (m,2H), 6,03 (s, 2H), 6,04 (s, 2H), 6,95 (m, 4H Ar), 7,09 (s, 1H), 7,34 (d, 2H, J = 8,99 Hz).
**Composé 1e** (base libre), δ (ppm) : 2,85 (dd, 1H, J = 12,47, 8,89 Hz), 3,04 (dd, 1H, J = 12,63, 3,02 Hz), 3,93 (d, 2H, J = 5,18 Hz), 4,07 (s, 2H), 4,23 (m, 1H), 5,85 (m, 1H Ar), 6,03 (s, 2H), 6,53 (m, 2H Ar), 6,92 (d, 1H Ar, J = 8,0 Hz), 7,02 (dd, 1H Ar, J = 8,0, 1,58 Hz), 7,19 (m, 2H Ar).
**Composé 1f** (base libre), δ (ppm) : 2,58 (t, 2H, J = 5,64 Hz), 3,62 (s, 2H), 3,89 (m, 1H), 3,97 (dd, 1H, J= 15,59, 5,98 Hz), 4,11 (dd, 1H, J = 15,14, 4,0 Hz), 4,99 (b, 1H), 5,94 (s, 2H), 6,78 (m, 2H), 6,89 (s, 1H), 7,39 (ddd, 1H, J = 8,29, 2,47, 0,95 Hz), 7,55 (dd, 1H, J = 6,93, 1,05 Hz).
**Composé 1g** (base libre), δ (ppm) : 2,55 (m, 2H), 3,61 (s, 2H), 3,89-4,05 (m, 3H), 4,97 (s, 1H), 5,95 (s, 2H), 6,72-6,78 (m, 2H), 6,88 (s, 1H), 7,24-7,50 (m, 4H).
**Composé 1h** (base libre), δ (ppm) : 2,58 (m, 2H), 3,61 (s, 2H), 3,90-4,05 (m, 3H), 4,97 (s, 1H), 5,94 (s, 2H), 6,72-6,78 (m, 2H), 6,89 (s, 1H), 7,20-7,27 (m, 3H), 7,49 (t, 1H, J = 7,69 Hz).

### EXEMPLE 2

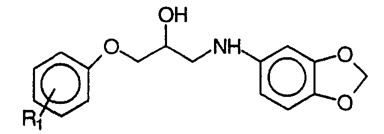

### N-(benzodioxole-5-yl)-2-hydroxy-3-(alkoxyphénoxy)-propylamine

De manière analogue à celle de l'Exemple 1, les composés selon l'exemple 2 sont préparés à partir d'un époxyde du commerce.

Les propriétés chimiques et les spectres RMN de ces produits sont illustrés ci-après :

**TABLEAU II**

| **Données chimiques des composés selon l'exemple 2** | | | | |
|---|---|---|---|---|
| **Composé** | **R**_{**1**} | **Rdt. %** | **Solvant** | **Pt de fusion °C** |
| 2a | H | 45 | EtOAc/cyclohex (2/8) | 80 - 81 |
| 2b | p-OMe | 65 | Et₂O/cyclohex | 80 - 81 |

### Spectres RMN ¹H (δ) des composés 2

**Composé 2a** (base libre), δ (ppm) : 3,22 (dd, 1H, J = 12,77, 7,02 Hz), 3,37 (dd, 1H, J = 12,76, 4,21 Hz), 4,05 (m, 2H), 4,22 (m, 1H), 5,86 (s, 2H), 6,11 (dd, 1H Ar, J = 8,26, 2,27 Hz), 6,32 (d, 1H Ar, J = 2,25 Hz), 6,66 (d, 1H Ar, J = 8,26 Hz) 6,96 (m, 3H, Ar), 7,30 (m, 2H, Ar).
**Composé 2b** (base libre), δ (ppm) : 3,21 (dd, 1H, J = 12,75, 7,07 Hz), 3,35 (dd, 1H, J = 12,73, 4,22 Hz), 3,78 (s, 3H), 3,97 (dd, 1H, J = 9,55, 6,06 Hz), 4,03 (dd, 1H, J = 11,49, 6,18 Hz), 4,20 (m, 1H), 5,86 (s, 2H), 6,11 (dd, 1H Ar, J = 8,31, 2,34 Hz), 6,31 (d, 1H Ar, J = 2,30 Hz), 6,66 (d, 1H Ar, J = 8,28 Hz), 6,85 (s, 4H Ar).

### EXEMPLE 3

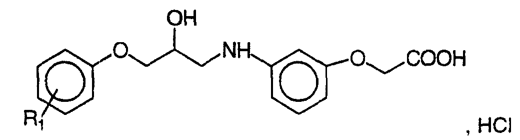

### Acide 3-[(3-phénoxy-2-hydroxypropyl)amino]phénoxy-acétique, sel d'hydrochlorure (R₁ = H)

Un mélange de 3 g (21,56 mmoles) de 3-nitro-phénol, 4,3 g (22 mmoles) de tert-butylbromoacétate, 3,2 g de carbonate de potassium anhydre et 50 ml d'acétone est agité une nuit à température ambiante. Le mélange est filtré, lavé à l'acétone et aussi bien le filtrat que le produit de lavage sont évaporés jusqu'à l'obtention d'une huile jaune. L'huile est purifiée sur colonne de chromatographie de gel de silice, éluée avec un mélange EtOAc/cyclohexane (50/50) pour obtenir 5 g (92 %) de tert-butyl-3-nitrophénoxyacétate (huile incolore). On ajoute à une solution de 4,8 g (18,95 mmoles) de tert-butyl-3-nitrophénoxyacétate dans 20 ml d'éthanol, 2 g de Pd/C (10 %) et le mélange est hydrogéné une nuit à une atmosphère. Le catalyseur est éliminé par filtration. Le solvant est évaporé pour obtenir 3,38 g (80 %) de tert-butyl-3-amidophénoxyacétate, qui est chromatographié sur une colonne de gel de silice en utilisant comme éluant un mélange EtOAc/cyclohexane (50/50). Un mélange de l'amine obtenue (0,5 g, 2,24 mmoles) et de 1,2-époxy-3-phénoxypropane est chauffé à 70°C pendant une nuit. Après refroidissement, le produit est lavé avec de l'éthanol et le précipité est filtré pour donner 0,6 g de tert-butyl ester. 0,6 g de cet ester et 15 ml d'HCl 6N sont mélangés une nuit à 80°C. Le solvant est évaporé et le résidu est lavé avec un mélange EtOH, CH₃CN puis recristallisé à partir du CH₃CN pour donner 0,36 g de sel dont les caractéristiques sont les suivantes : point de fusion 138-139°C ; RMN de ¹H (DMSO-d₆) δ 3,82 (m, 2H), 3,96 (m, 4H), 4,06 (m, 1H), 4,72 (s, 2H), 6,88-7,00 (m, 5H Ar), 7,24-7,45 (m, 4H Ar).

### EXEMPLE 4

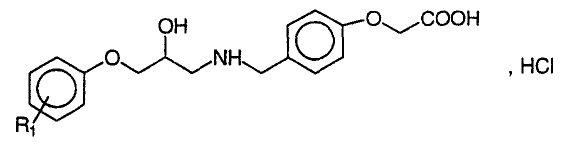

### Acide 4-[(3-phénoxy-2-hydroxypropyl)amidométhyl]phén-oxyacétique, sel d'hydrochlorure (R₁ = H)

Un mélange de 22,16 g (0,186 mole) de 4-cyanophénol, 32,07 g (0,192 mole) d'éthylbromoacétate, 27,6 g (0,199 mole) de K₂CO₃ et d'acétone (250 ml) est agité à température ambiante pendant 12 heures. Après filtration, le solvant est évaporé sous vide et le résidu réparti entre une phase organique (éthylacétate) et une phase aqueuse (eau). La couche organique est séchée (MgSO₄) et concentrée sous vide pour donner 36,42 g (95 %) d'éthyl-4-cyanophénoxy-acétate (solide blanc, point de fusion : 49-50°C.

On ajoute 1,5 g de Pd/C (10 %) à une solution de l'ester obtenu (4,1 g, 20 mmoles) dans de l'éthanol (80 ml) et le mélange est hydrogéné sous une pression de 50 bars à température ambiante pendant 16 heures. Le catalyseur est éliminé par filtration (Célite^{R}) et le filtrat est concentré sous vide, pour donner un mélange de 38 % de dérivé monobenzylamine et de 42 % de dérivé dibenzylamine, qui est chromatographié sur gel de silice en utilisant comme éluant, un mélange EtOAc/MeOH : (8/2).

0,4 g (1,9 mmole) d'amine 16 (n = 1 et R = Et) est dissous dans du DMF (5 ml) contenant un équivalent (0,3 g, 2 mmoles) de (±)-1,2-époxy-3-phénoxypropane et chauffé une nuit à 80°C. Après refroidissement, le solvant est éliminé sous vide et le résidu huileux est trituré avec Et₂O, filtré et lavé avec EtOH et MeOH pour donner 0,25 g (37 %) d'acide éthyl-4-[3-phénoxy-2-hydroxypropyl)amidométhyl]phénoxyacétique, un ester sous la forme d'un solide blanc, de point de fusion : 120°C, qui est hydrolysé avec HCl 6N, comme précisé à l'Exemple 3, pour donner le dérivé recherché, sous la forme d'un solide blanc.

D'autres composés sont préparés selon la technique décrite dans l'Exemple 1 et présentent les caractéristiques ci-dessous :

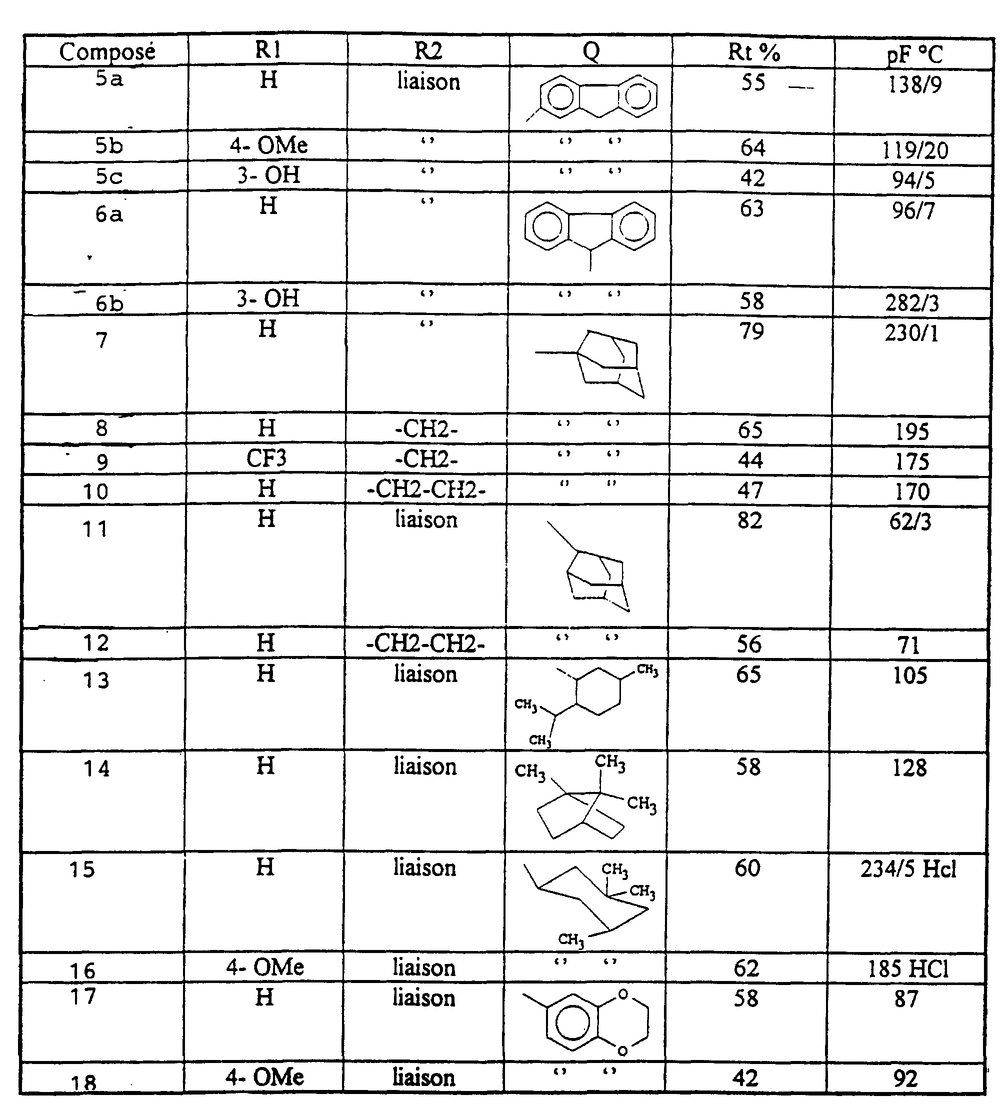

### Spectres RMN ¹H (δ) des composés 5 à 8

**Composé 5a** (maléate), δ (ppm) : 3,17 (dd, 1H, J = 11,97, 5,21 Hz), 3,31 (dd, 1H, J = 12,0, 4,9 Hz), 3,74 (s, 2H), 4,01 (m, 3H), 6,25 (s, 2H), 6,68 (dd, 1H Ar, J = 8,27, 2,06 Hz), 6,88 (m, 4H Ar), 7,10 (m, 1H Ar), 7,27 (m, 3H Ar), 7,43 (d, 1H Ar, J = 7,16 Hz), 7,58 (m, 2H Ar).
**Composé 5b** (base libre), δ (ppm) : 3,16 (m, 1H), 3,30 (m, 1H), 3,69 (s, 3H), 3,73 (s, 2H), 3,90 (m, 3H), 5,16 (d, 1H, J = 4,84 Hz), 5,72 (t, 1H NH, J = 5,79 Hz), 6,66 (dd, 1H, J = 8,27, 2,01 Hz), 6,89 (m, 5H Ar), 7,10 (dt, 1H Ar, J = 7,36, 1,09 Hz), 7,25 (dt, 1H Ar, J = 7,43, 0,8 Hz), 7,42 (d, 1H, J = 7,26 Hz), 7,54 (d, 1H Ar, J = 8,27 Hz), 7,60 (d, 1H Ar, J = 7,14 Hz).
**Composé 5c** (maléate), δ (ppm) : 3,58 (m, 2H), 3,94 (m, 4H), 4,11 (m, 1H), 6,17 (s, 2H), 6,37 (s, 1H Ar), 6,56 (d, 2H, J = 7,96 Hz), 6,81 (d, 1H Ar, J = 7,58 Hz), 6,97-7,23 (m, 4H Ar), 7,42 (m, 1H Ar), 7,57 (m, 2H Ar).
**Composé 6a** (HCl), δ (ppm) : 3,54 (m, 2H), 3,85 (m, 3H), 4,15 (m, 1H), 5,64 (s, 1H); 6,75-6,92 (m, 4H Ar), 7,17-7,25 (m, 2H), 7,38-7,56 (m, 4H Ar), 7,96 (m, 2H Ar), 8,14 (m, 1H Ar).
**Composé 7** (HCl), δ (ppm) : 1,61 (m, 5H), 1,91 (m, 6H), 2,10 (m, 4H), 2,94 (m, 1H), 3,12 (m, 1H), 3,99 (dd, 2H, J = 5,08 Hz), 4,22 (m, 1H), 6,90-6,97 (m, 3H Ar), 7,29 (t, 2H Ar, J = 8,29 Hz).
**Composé 8** (HCl), δ (ppm) : 1,59-1,92 (m, 10H), 2,41 (m, 4H), 3,62 (m, 3H), 4,03 (m, 2H), 4,56 (m, 1H), 6,92-6,99 (m, 3H Ar), 7,29 (t, 2H Ar, J = 7,31 Hz).

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Composés qui répondent à la formule générale I suivante : 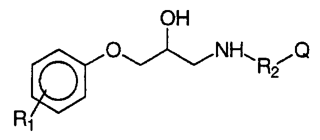 dans laquelle :
R₁, substituant du groupe phényle, en position 2, 3 ou 4, représente un atome d'hydrogène, un atome d'halogène ou l'un des groupes suivants : hydroxyle, alkyle inférieur en C₁-C₁₀, notamment choisi parmi les groupes méthyle, éthyle, propyle, isopropyle, butyle, tertiobutyle ; alkyloxy en C₁-C₁₀ ; benzyloxy ; nitro ; cyano ; trifluorométhyle ; amino éventuellement substitué (mono ou di-substitué) par 1 ou 2 radicaux alkyles inférieurs, tels que précisés ci-dessus,
R₂ représente l'un des groupes suivants : -CH₂-, -CH₂-CH₂-, -CH(CH₃)-CH₂-, -C(CH₃)=CH-, -C(CH₃)₂-CH₂- ou une liaison ;
Q représente :
(i) un radical phényle 3,4-disubstitué alkylène dioxy déterminant avec le radical phényle, un motif benzodioxane non substitué, un motif benzodioxol non substitué, ou un motif benzodioxol 2-substitué par deux radicaux R₃ et R₄, conformément à la formule suivante : 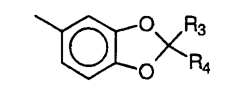 dans laquelle R₃ et R₄ sont sélectionnés de manière indépendante dans le groupe constitué par un atome d'hydrogène ou un groupe hydroxyméthyle,
(ii) un radical phényle 3 et/ou 4-substitué, conformément à la formule II suivante 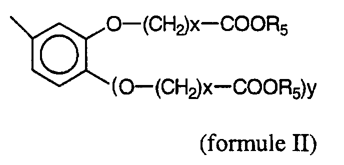 dans laquelle
R₅ représente un atome d'hydrogène ou un radical alkyle inférieur en C₁-C₆, linéaire ou ramifié,
x est un nombre entier compris entre 1 et 3
y est 0 ou 1,
(iii) un hydrocarbure polycyclique condensé comprenant au moins deux cycles condensés et choisis parmi les motifs suivants : indène, indacène, naphtalène, azulène, biphénylène, acénaphtylène, fluorène, phénalène, phénanthrène, anthracène,
(iv) un système hydrocarboné cyclique, éventuellement ponté, et constituant un cycloalkane comprenant 1, 2 ou 3 cycles, comportant éventuellement des substituants choisis parmi les groupes méthyle, éthyle, isopropyle, propyle, butyle, tertiobutyle, le cycle principal comportant 5 ou 6 chaînons.

2. Composé selon la revendication 1, **caractérisé en ce qu'**il répond à la formule suivante : 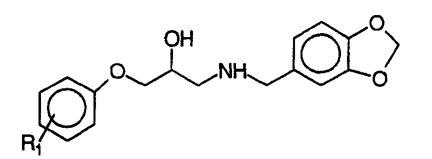 dans laquelle R₁ représente un atome d'hydrogène ; un méthoxy ou un atome de chlore en position para ; un atome de chlore, un groupe hydroxy, un groupe nitro, un groupe cyano ou un groupe trifluorométhyle en position méta.

3. Composé selon la revendication 1, **caractérisé en ce qu'**il répond à la formule suivante : 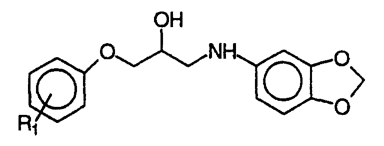 dans laquelle R₁ représente un atome d'hydrogène ou un groupe méthoxy en para.

4. Composé selon la revendication 1, **caractérisé en ce qu'**il répond à la formule suivante : 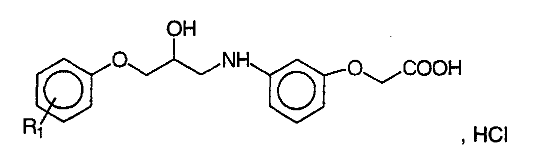 dans laquelle R₁ représente un atome d'hydrogène.

5. Composé selon la revendication 1, **caractérisé en ce qu'**il répond à la formule suivante : 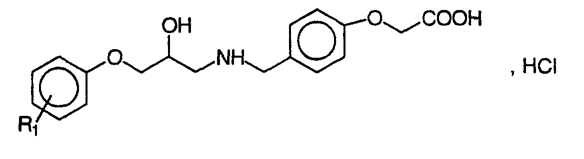 dans laquelle R₁ représente un atome d'hydrogène.

6. Composés selon la revendication 1, **caractérisés en ce que** :
R₁ représente un atome d'hydrogène, un 4-O méthyle, un 3-OH, un groupe trifluorométhyle,
R₂ représente une liaison, ou l'un des groupes suivants : -CH₂- ou -CH₂-CH₂- et
Q représente un groupe fluorène ou un système hydrocarboné cyclique.

7. Procédé de préparation d'un composé de formule (I) selon l'une quelconque des revendications 1 à 6, **caractérisé en ce qu'**il comprend la réaction :
- d'un composé phénoxyépoxypropane de formule III 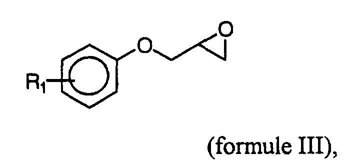
dans laquelle R₁ a la signification ci-dessus, avec
- une amine primaire répondant à la formule (IV) 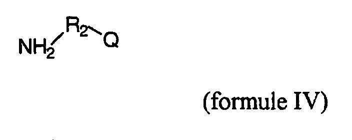
dans laquelle R₂ et Q ont les significations ci-dessus.

8. Procédé selon la revendication 7, **caractérisé en ce que** les intermédiaires de formule (III) et de formule (IV) NH₂-R₂-Q sont couplés par chauffage (sous forme brut ou en solution dans un solvant polaire tel que le diméthylformamide anhydre), pendant 12 à 14 heures, à une température comprise entre 70 et 100°C, pour fournir des composés de formule (I).

9. Procédé selon la revendication 7 ou la revendication 8, **caractérisé en ce que** lorsque lesdits composés de formule (I) comportent une ou deux fonctions esters, lesdits composés estérifiés sont mis en contact respectivement avec HCl 6N à 80°C pendant 12 heures et avec NaOH 1N à température ambiante pendant 12 à 48 heures, pour obtenir le sel d'acide carboxylique correspondant.

10. Médicament, **caractérisé en ce qu'**il comprend au moins un des composés selon l'une quelconque des revendications 1 à 6, à l'état pur ou à l'état de sels avec des acides ou des bases pharmaceutiquement acceptables et au moins un véhicule pharmaceutiquement acceptable.

11. Médicament, **caractérisé en ce qu'**il comprend au moins un des composés selon l'une quelconque des revendications 1 à 6, à l'état pur ou à l'état de sels avec des acides ou des bases pharmaceutiquement acceptables et au moins un véhicule pharmaceutiquement acceptable, destiné à combattre les problèmes de poids et/ou les troubles du fonctionnement métabolique.

12. Additif alimentaire dans l'élevage animal, **caractérisé en ce qu'**il comprend au moins un composé selon l'une quelconque des revendications 1 à 6.

## Claims

1. Compounds of general formula I below: 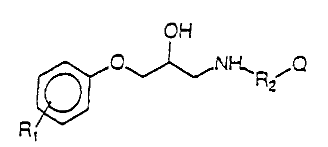 in which:
R₁, a substituent in the 2-, 3- or 4-position of the phenyl group, is a hydrogen atom, a halogen atom or one of the following groups: hydroxyl; C₁-C₁₀ lower alkyl selected in particular from methyl, ethyl, propyl, isopropyl, butyl and tert-butyl groups; C₁-C₁₀-alkoxy; benzyloxy; nitro; cyano; trifluoromethyl; or amino optionally substituted (monosubstituted or disubstituted) by 1 or 2 lower alkyl radicals as defined above;
R₂ is one of the following groups: -CH₂-, -CH₂-CH₂-, -CH(CH₃)-CH₂-, -C(CH₃)=CH-, -C(CH₃)₂-CH₂-, or a bond; and
Q is:
(i) a phenyl radical 3,4-disubstituted by alkylenedioxy, which forms, with the phenyl radical, an unsubstituted benzodioxane unit, an unsubstituted benzodioxole unit or a benzodioxole unit 2-substituted by two radicals R₃ and R₄ according to the following formula: 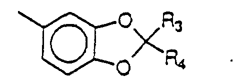 in which R₃ and R₄ are independently selected from the group comprising a hydrogen atom and a hydroxymethyl group;
(ii) a 3- and/or 4-substituted phenyl radical of formula II below: 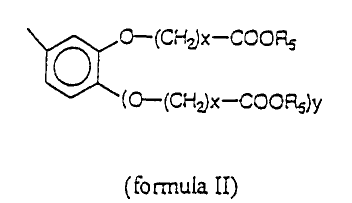 in which:
R₅ is a hydrogen atom or a linear or branched C₁-C₆ lower alkyl radical;
x is an integer between 1 and 3; and
y is 0 or 1;
(iii) a fused polycyclic hydrocarbon comprising at least two fused rings and selected from the following units: indene, indacene, naphthalene, azulene, biphenylene, acenaphthylene, fluorene, phenalene, phenanthrene and anthracene; or
(iv) an optionally bridged, cyclic hydrocarbon system which consists of a cycloalkane comprising 1, 2 or 3 rings optionally containing substituents selected from methyl, ethyl, isopropyl, propyl, butyl and tert-butyl groups, the main ring containing 5 or 6 members.

2. Compound according to Claim 1, **characterized in that** it has the following formula: 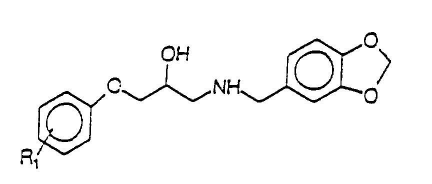 in which R₁ is a hydrogen atom, a methoxy or a chlorine atom in the para position, or a chlorine atom, a hydroxyl group, a nitro group, a cyano group or a trifluoromethyl group in the meta position.

3. Compound according to Claim 1, **characterized in that** it has the following formula: 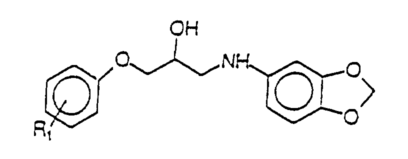 in which R₁ is a hydrogen atom or a methoxy group in the para position.

4. Compound according to Claim 1, **characterized in that** it has the following formula: 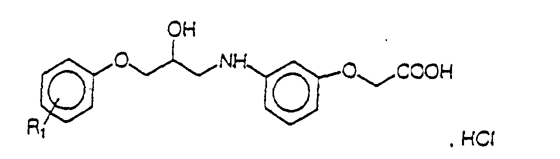 in which R₁ is a hydrogen atom.

5. Compound according to Claim 1, **characterized in that** it has the following formula: 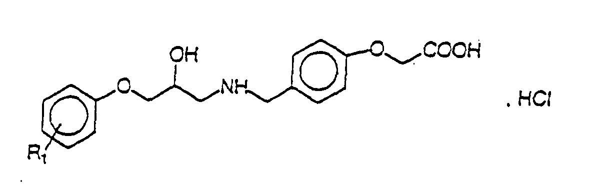 in which R₁ is a hydrogen atom

6. Compounds according to Claim 1, **characterized in that**:
R₁ is a hydrogen atom, a 4-O-methyl, a 3-OH or a trifluoromethyl group;
R₂ is a bond or one of the following groups: -CH2- or -CH₂-CH₂-; and
Q is a fluorene group or a cyclic hydrocarbon system.

7. Process for the preparation of a compound of formula (I) according to any one of Claims 1 to 6, **characterized in that** it comprises reacting:
- a phenoxyepoxypropane compound of formula III: 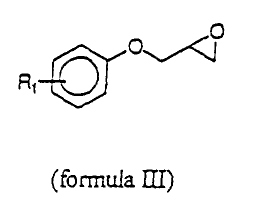 in which R₁ is as defined above, with
- a primary amine of formula (IV): 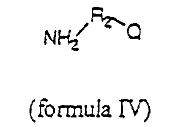 in which R₂ and Q are as defined above.

8. Process according to Claim 7, **characterized in that** the intermediates of formula (III) and of formula (IV), NH₂-R₂-Q, are coupled by heating (in the crude form or dissolved in a polar solvent such as anhydrous dimethylformamide) for 12 to 14 hours, at a temperature between 70 and 100°C, to give compounds of formula (I).

9. Process according to Claim 7 or Claim 8, **characterized in that** if said compounds of formula (I) contain one or two ester groups, said esterified compounds are brought into contact respectively with 6 N HCl at 80°C for 12 hours and with 1 N NaOH at room temperature for 12 to 48 hours to give the corresponding carboxylic acid salt.

10. Drug, **characterized in that** it comprises at least one of the compounds according to any one of Claims 1 to 6, in the pure state or in the form of salts with pharmaceutically acceptable acids or bases, and at least one pharmaceutically acceptable vehicle.

11. Drug, **characterized in that** it comprises at least one of the compounds according to any one of Claims 1 to 6, in the pure state or in the form of salts with pharmaceutically acceptable acids or bases, and at least one pharmaceutically acceptable vehicle, for combating weight problems and/or metabolic function disorders.

12. Feed additive for animal rearing, **characterized in that** it comprises at least one compound according to any one of Claims 1 to 6.

## Patentansprüche

1. Verbindungen der folgenden allgemeinen Formel I: 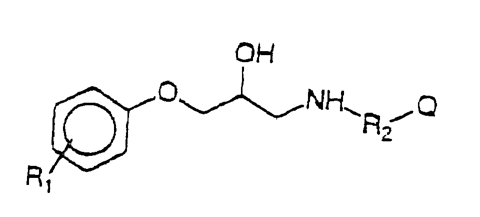 worin:
der Substituent R₁ der Phenyl-Gruppe in Position 2, 3 oder 4 für ein Wasserstoffatom, ein Halogenaton oder eine der folgenden Gruppen: Hydroxyl, C₁-C₁₀ Niedrigalkyl, insbesondere ausgewählt aus den Gruppen Methyl, Ethyl, Propyl, Isopropyl, Butyl, tert-Butyl; C₁-C₁₀ Alkoxy; Benzyloxy; Nitro; Cyano; Trifluormethyl; gegebenenfalls durch ein oder zwei nachstehende präzisierte (mono oder disubstituierte ) Alkylreste substituiertes Amino steht,
R₂ für eine der folgenden Gruppen steht: -CH₂-, -CH₂-CH₂-, -C(CH₃)-CH₂-, -C(CH₃)=CH-, -C(CH₃)₂-CH₂- oder eine Einfachbindung
Q für:
(i) einen Phenylrest , welcher mit einer Alkylendioxygruppe, die mit dem Phenylrest verknüpft ist, 3,4-disubstituiert ist, eine unsubstituierte Benzodioxangruppe, eine unsubstituierte Benzodioxolgruppe oder eine durch zwei Reste R₃ und R₄ 2-substituierte Benzodioxolgruppe der folgenden Formel: 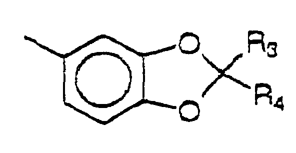 worin R₃ und R₄ unabhängig aus der Gruppe bestehend aus einem Wasserstoffatom oder einer Hydroxymethylgruppe ausgewählt sind,
(ii) einen 3-und/oder 4-substituierten Phenylrest der folgenden Formel II 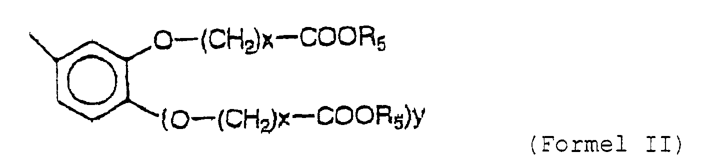 worin
R₅ für ein Wasserstoffatom oder eine geradkettige oder verzweigtkettige C₁-C₆ Niedrigalkylgruppe steht,
x eine ganze Zahl von 1 bis 3 bedeutet
y 0 oder 1 ist
(iii) einen kondensierten polyzyklischen Kohlenwasserstoff umfassend mindestens zwei kondensierte Ringe ausgewählt aus den folgenden Gruppen: Inden, Indacen, Naphthalin, Azulen, Biphenylen, Acenaphthen, Fluoren, Phenalen, Phenanthren, Anthracen,
(iv) ein gegebenenfalls verbrücktes cyklisches Kohlenwasserstoff-System, welches eine Cykloalkan umfassend 1, 2 oder 3 Ringe bildet, und gegebenenfalls Substituenten ausgewählt aus den Gruppen Methyl, Ethyl, Propyl, Isopropyl, Butyl, tert-Butyl trägt, wobei der Hauptring 5 oder 6 Kettenglieder aufweist, steht.

2. Verbindung nach Anspruch 1 **dadurch gekennzeichnet, dass** sie der folgenden Formel entspricht: 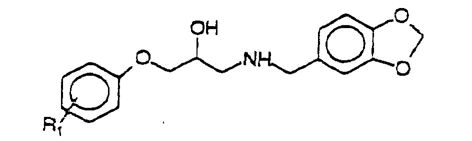 worin R₁ für einen Wasserstoffatom; Methoxy oder ein Chloratom in para-Position; ein Chloratom, eine Hydroxygruppe, eine Nitrogruppe, eine Cyanogruppe oder eine Trifluormethylgruppe in meta-Position steht.

3. Verbindung nach Anspruch 1 **dadurch gekennzeichnet, dass** sie der folgenden Formel entspricht: 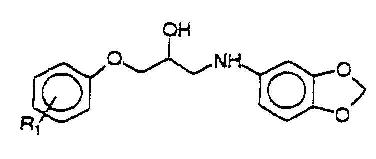 worin R₁ für ein Wasserstoffatom oder eine para-Methoxygruppe steht.

4. Verbindung nach Anspruch 1 **dadurch gekennzeichnet, dass** sie der folgenden Formel entspricht: 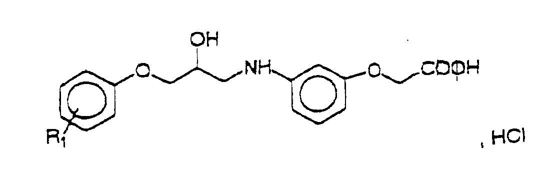 worin R₁ für ein Wasserstoffatom steht.

5. Verbindung nach Anspruch 1 **dadurch gekennzeichnet, dass** sie der folgenden Formel entspricht: 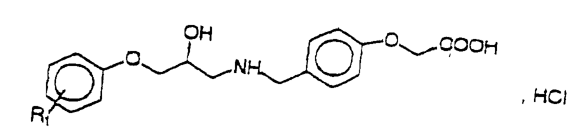 worin R₁ für ein Wasserstoffatom steht.

6. Verbindungen nach Anspruch 1 **dadurch gekennzeichnet, dass**
R₁ für ein Wasserstoffatom, 4-O-Methyl, 3-OH, eine Trifluormethylgruppe steht,
R₂ für eine Einfachbindung oder eine der folgenden Gruppen: - CH₂- oder -CH₂-CH₂- steht und
Q eine Fluorengruppe oder ein cyklisches Kohlenwasserstoff-System steht.

7. Verfahren zur Herstellung einer Verbindung der Formel (I) nach einem der Ansprüche 1 bis 6 **dadurch gekennzeichnet, dass** es die Umsetzung:
- einer Phenoxyepoxypropan-Verbindung der Formel III 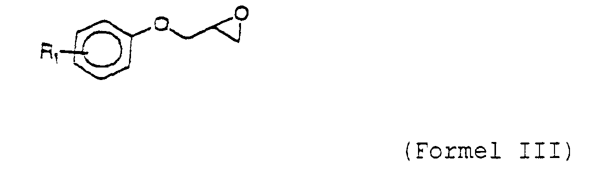
worin R₁ die oben angegebene Bedeutung hat, mit
- einem primären Amin der Formel (IV) 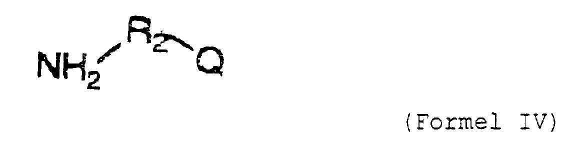
worin R₂ und Q die oben angegebenen Bedeutungen haben, umfasst.

8. Verfahren nach Anspruch 7 **dadurch gekennzeichnet, dass** die Zwischenstufen der Formel (III) und der Formel (IV) NH₂-R₂-Q durch Erhitzen (in Substanz oder in Lösung in einem polaren Lösungsmittel wie wasserfreiem Dimethylformamid) über 12 bis 14 Stunden bei einer Temperatur von 70 bis 100°C verknüpft werden, um Verbindungen der Formel (I) herzustellen.

9. Verfahren nach Anspruch 7 oder 8 **dadurch gekennzeichnet, dass** die Verbindungen der Formel (I) eine oder zwei Esterfunktionen tragen, wobei die veresterten Verbindungen mit 6N HCl bei 80°C über 12 Stunden bzw. 1N NaOH bei Raumtemperatur über 12 bis 48 Stunden kontaktiert werden um das entsprechende Carbonsäuresalz zu erhalten.

10. Arzneimittel **dadurch gekennzeichnet, dass** es mindestens eine der Verbindungen nach einem der Ansprüche 1 bis 6 als solche oder als Salze mit pharmazeutisch annehmbaren Säuren oder Basen und mindestens einen pharmazeutisch annehmbaren Träger umfasst.

11. Arzneimittel **dadurch gekennzeichnet, dass** es mindestens eine der Verbindungen nach einem der Ansprüche 1 bis 6 als solche oder als Salze mit pharmazeutisch annehmbaren Säuren oder Basen und mindestens einen pharmazeutisch annehmbaren Träger umfasst und bestimmt ist zur Behandlung von Gewichtsproblemen und/oder Störungen des Stoffwechsels.

12. Futterzusatz für die Tierzucht **dadurch gekennzeichnet, dass** es mindestens eine Verbindung nach einem der Ansprüche 1 bis 6 umfasst.
